# EUROPEAN PATENT APPLICATION

(11) **EP 4 212 159 A1**
(43) Date of publication of application: **19.07.2023**
(21) Application number: 21872967.1
(22) Date of filing: 27.09.2021
(51) Int. Cl.: A61K 31/454, A61P 17/00

(54) **USE OF SPHINGOSINE-1-PHOSPHATE RECEPTOR AGONIST**

(30) Priority: 28.09.2020 KR 20200125583
(71) Applicant: Lg Chem, Ltd., Seoul 07336 (KR)
(72) Inventor: KIM, Ki Chan, Seoul 07796 (KR); KIM, Tae Hun, Seoul 07796 (KR)
(74) Representative: Hoffmann Eitle
(86) International application number: PCT/KR2021/013097
(87) International publication number: WO 2022/065939

(57) **Abstract**

The present invention relates to use of a compound of chemical formula 1 or a pharmaceutically acceptable salt thereof for the prevention or treatment of atopic dermatitis.

## Description

### TECHNICAL FIELD

The present invention relates to the use of a compound of Formula 1 or a pharmaceutically acceptable salt thereof for the purpose of preventing or treating atopic dermatitis: 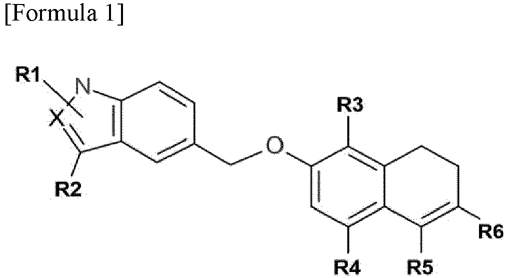 wherein X, R1, R2, R3, R4, R5 and R6 are the same as defined herein.

### BACKGROUND ART

Sphingosine-1-phosphate (S1P) is produced via an intracellular ceramide pathway, in which ceramide is the starting material. Ceramide is produced via two pathways, the first of which is a *de novo* biosynthetic pathway. Ceramide is also produced by the degradation of sphingomyelin, a cell membrane constituent, in a cell. The S1P level in each tissue is controlled by two biosynthetic sphingosine kinases (SphKs) and two biodegradable S1P phosphatases (S1P lyase and lysophospholipid phosphatases). S1P-which is produced via phosphorylation of sphingosine by sphingosine kinase-is known to mediate various cellular responses, such as cell proliferation, cytoskeletal organization and migration, adherence- and tight junction assembly, and morphogenesis. S1P exists as a combined form with plasma protein including albumin at high level (100 - 1,000 nM) in plasma, while it is at a low level in tissues.

S1P binds with S1P receptor, a G-protein coupled receptor, to show various biological functions. As S1P receptor sub-types, S1P1 to S1P5 are known up to now and are named endothelial differentiation gene (EDG) receptors 1, 5, 3, 6 and 8, respectively. The S1P receptors are known to be involved in various biological functions such as leukocyte recirculation, neural cell proliferation, morphological changes, migration, endothelial function, vasoregulation and cardiovascular development.

In recent years, many studies have found that the S1P signaling process via these receptors plays an important role in a series of responses related to multiple sclerosis including inflammation response and the repair process, and a non-selective S1P1 agonist was actually approved as a therapeutic agent for multiple sclerosis. S1P receptors are extensively expressed in many cells related to the induction of multiple sclerosis. Especially, S1P1 receptor plays a major role in the immune system. S1P1 receptor is mainly expressed on the surface of lymphocytes such as T cell and B cell, and responds to S1P resulting in involvement in recirculation of lymphocytes. In normal condition, the S1P concentration is higher in body fluid than in lymphoid tissue, and therefore lymphocytes leave lymphoid tissue by the difference of S1P concentration to circulate after efferent lymph circulates. However, if S1P1 receptor in lymphocytes is down-regulated by S1P1 agonist, the egress of lymphocytes from lymphoid tissue does not occur, resulting in reduced infiltration of autoaggressive lymphocytes which cause inflammation and tissue damage in the central nervous system (CNS). As a result, a therapeutic effect on multiple sclerosis is obtained. Fingolimod-which is a non-selective S1P1 agonist-has been approved as an oral medication for the treatment of multiple sclerosis. When it binds at S1P1 receptor to be activated, the receptor becomes degraded or internalized from the surface of lymphocytes ironically, and thus it acts as a functional S1P1 antagonism.

With respect to such S1P receptor, International Publication No. WO 2014/129796 (publication date: August 28, 2014) discloses compounds effective as S1P receptor agonists.

Meanwhile, atopic dermatitis is a chronic recurrent skin eczema disease, and although the cause has not been clearly identified until now, it has been reported that immune-related factors overreact due to the action of external antigens. Drugs used for atopic dermatitis are mainly immunosuppressants, topical steroids, antihistaminic agents, etc. However, there are side effects according to the use of such drugs or limitations in improvement of atopic dermatitis. As a result, there is a continuous need for new drugs that can more effectively prevent or treat atopic dermatitis.

### DISCLOSURE OF INVENTION

### TECHNICAL PROBLEM

An object of the present invention is to provide the use of a compound of the following Formula 1 or a pharmaceutically acceptable salt thereof in the prevention or treatment of atopic dermatitis: 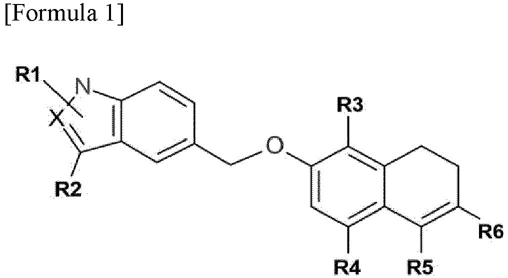 wherein X, R1, R2, R3, R4, R5 and R6 are the same as defined herein.

### SOLUTION TO PROBLEM

The present invention provides a medicament for the prevention or treatment of atopic dermatitis comprising a therapeutically effective amount of a compound of the following Formula 1 or a pharmaceutically acceptable salt thereof: 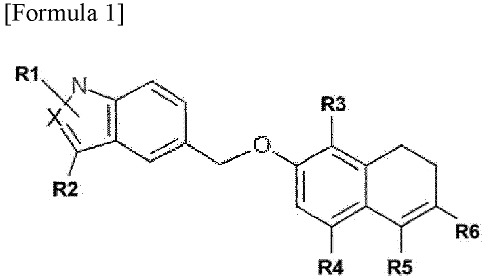 wherein
X is carbon or nitrogen;
R1 is hydrogen or alkyl;
R2 is hydrogen, alkyl, halogen, CN, CF₃ or COCF₃;
R3 and R4 are each independently hydrogen, alkyl, halogen, haloalkyl or alkoxyalkyl;
R5 is hydrogen, alkyl or halogen;
R6 is 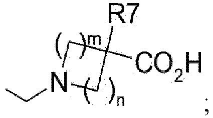
R7 is hydrogen or alkyl; and
m and n are each independently 0, 1, 2 or 3.

In addition, the present invention provides a pharmaceutical composition for the prevention or treatment of atopic dermatitis comprising a therapeutically effective amount of the compound of Formula 1 or a pharmaceutically acceptable salt thereof, together with a pharmaceutically acceptable carrier.

In addition, the present invention provides a method for the prevention or treatment of atopic dermatitis comprising administering a therapeutically effective amount of the compound of Formula 1 or a pharmaceutically acceptable salt thereof to a subject in need thereof.

In addition, the present invention provides use of the compound of Formula 1 or a pharmaceutically acceptable salt thereof for the prevention or treatment of atopic dermatitis.

The present invention is described in detail hereinafter.

According to one aspect to the present invention, there is provided a medicament for the prevention or treatment of atopic dermatitis comprising a therapeutically effective amount of the compound of Formula 1 or a pharmaceutically acceptable salt thereof.

According to another aspect to the present invention, there is provided a pharmaceutical composition for the prevention or treatment of atopic dermatitis comprising a therapeutically effective amount of the compound of Formula 1 or a pharmaceutically acceptable salt thereof, together with a pharmaceutically acceptable carri er.

The method for preparing the compound of Formula 1 is described in detail in International Publication No. WO 2014/129796 A1, and the document is incorporated herein by reference.

In one embodiment according to the present invention, in Formula 1
X is CH or N;
R1 is hydrogen or C₁-C₅ alkyl;
R2 is hydrogen, C₁-C₅ alkyl, halogen, CN, CF₃ or COCF₃;
R3 and R4 are each independently hydrogen, C₁-C₅ alkyl, halogen, halo-C₁-C₅ alkyl or C₁-C₅ alkoxy-C₁-C₅ alkyl;
R5 is hydrogen, C₁-C₅ alkyl or halogen;
R6 is 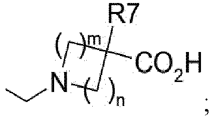
R7 is hydrogen or C₁-C₅ alkyl; and
m and n are each independently 0, 1, 2 or 3.

In another embodiment according to the present invention, the compound of Formula 1 is 1-[1-chloro-6-(3-chloro-1-isopropyl-1H-indazol-5-ylmethoxy)-3,4-dihydro-naphthalen-2-ylmethyl]-piperidine-4-carboxylic acid of the following Formula 2:

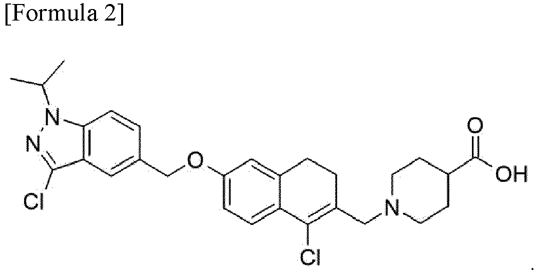

In another embodiment according to the present invention, the pharmaceutically acceptable salt includes, for example, acid-addition salts which are formed from nontoxic acid addition salts containing pharmaceutically acceptable anions, such as inorganic acids such as hydrochloric acid, sulfuric acid, nitric acid, phosphoric acid, hydrobromic acid, hydroiodic acid; organic acids such as tartaric acid, formic acid, citric acid, acetic acid, trichloroacetic acid, trifluoroacetic acid, gluconic acid, benzoic acid, lactic acid, fumaric acid, maleic acid; or sulfonic acids such as methanesulfonic acid, benzenesulfonic acid, p-toluenesulfonic acid or naphthalenesulfonic acid, but are not limited thereto.

In another embodiment according to the present invention, a "therapeutically effective amount" for an individual subject refers to an amount sufficient to achieve the above pharmacological effect-i.e., the therapeutic effect as described above. The amount of the compound may vary depending on the condition and severity of the subject, the mode of administration and the age of the subject to be treated, but could be determined by persons of ordinary skill in the art based on their knowledge.

In another embodiment according to the present invention, the therapeutically effective dosage of the compound of Formula 1 is, for example, typically in the range of about 0.1 to 500 mg per day according to the frequency and intensity of administration. A typical daily dose of intramuscular or intravenous administration for adults is in the range of about 0.1 to 300 mg per day which can be administered in divided unit dosages. Some patients need a higher daily dose.

In the present invention, a "pharmaceutical composition" may include other components such as carriers, diluents, excipients, etc., in addition to the active ingredient of the present invention. Accordingly, the pharmaceutical composition may include pharmaceutically acceptable carriers, diluents, excipients or combinations thereof, if necessary. The pharmaceutical composition facilitates the administration of compounds into the body. Various methods for administering the compounds include, but are not limited to, oral, injection, aerosol, parenteral and local administration.

Herein, a "carrier" means a compound that facilitates the addition of compounds into the cell or tissue. For example, dimethylsulfoxide (DMSO) is a conventional carrier facilitating the administration of many organic compounds into living cells or tissues.

Herein, a "diluent" means a compound that not only stabilizes a biologically active form but is diluted in solvent dissolving the compounds. A dissolved salt in buffer is used as a diluent in this field. A conventionally used buffer is a phosphate buffer saline mimicking salt form in body fluid. Since a buffer solution can control the pH of the solution at low concentration, a buffer diluent hardly modifies the biological activity of compounds.

Herein, "pharmaceutically acceptable" means such property that does not impair the biological activity and physical property of compounds.

The compounds according to the present invention can be formulated as various pharmaceutically administered dosage forms. In the preparation of the pharmaceutical composition of the present invention, an active component-specifically, the compound of Formula 1 or a pharmaceutically acceptable salt thereof-is mixed with selected pharmaceutically acceptable carriers considering the dosage form to be prepared. For example, the pharmaceutical composition of the present invention can be formulated as injections, oral preparations and the like, as needed.

The compound of the present invention can be formulated by conventional methods using known pharmaceutical carriers and excipients, and inserted into a unit or multi-unit containers. The formulations may be solution, suspension or emulsion in oil or aqueous solvent and include conventional dispersing agents, suspending agents or stabilizing agents. In addition, the compound may be, for example, dry powder form which is dissolved in sterilized pyrogen-free water before use. The compound of the present invention can be formulated into suppositories by using a conventional suppository base such as cocoa butter or other glycerides. Solid forms for oral administration include capsules, tablets, pills, powders and granules. Capsules and tablets are preferred. Tablets and pills are preferably enteric-coated. Solid forms are manufactured by mixing the compounds of the present invention with at least one carrier selected from inert diluents such as sucrose, lactose or starch, lubricants such as magnesium stearate, disintegrating agents, binders and the like. In addition, it may be formulated as a transdermal dosage form-for example, as a lotion, ointment, gel, cream, patch or spray.

Herein, the term "prevention" refers to reducing or eliminating the possibility of contracting a disease.

Herein, the term "treatment" is used to mean deterring, delaying or ameliorating the progress of diseases in a subject exhibiting symptoms of diseases.

### EFFECTS OF THE INVENTION

The medicament or pharmaceutical composition according to the present invention can effectively prevent or treat atopic dermatitis.

### BRIEF DESCRIPTION OF DRAWINGS

Figure 1 is a schematic diagram showing the process of inducing an animal model of atopic dermatitis.
Figure 2 is the results of measuring the reduction in ear thickness after administration of test substances in an animal model of atopic dermatitis.
Figure 3 is the results of measuring the reduction in ear weight after administration of test substances in an animal model of atopic dermatitis.
Figure 4 is the results of measuring the reduction in the blood level of IgE after administration of test substances in an animal model of atopic dermatitis.
Figure 5 is the results of measuring the reduction in epidermal thickness after administration of test substances in an animal model of atopic dermatitis.
Figure 6 is the results of measuring the reduction in mast cell accumulation after administration of test substances in an animal model of atopic dermatitis.
Figure 7 is the results of comparison with etrasimod as a comparative compound in an animal model of atopic dermatitis.

### MODE FOR THE INVENTION

Hereinafter, the present invention is explained in more detail with the following examples. However, it must be understood that the protection scope of the present invention is not limited to the examples.

### Preparation Example: Synthesis of 1-[1-chloro-6-(3-chloro-1-isopropvl-1H-indazol-5-ylmethoxy)-3,4-dihydro-naphthalen-2-ylmethyl]-piperidine-4-carboxylic acid

1-[1-Chloro-6-(3-chloro-1-isopropyl-1H-indazol-5-ylmethoxy)-3,4-dihydro-naphthalen-2-ylmethyl]-piperidine-4-carboxylic acid (hereinafter referred to as "Test Compound") was prepared according to the method disclosed in Example 153 of International Publication No. WO 2014/129796 A1.

### Example 1: Induction of animal model of atopic dermatitis

An animal model of atopic dermatitis was induced in mice (Balb/c) by the use of oxazolone. With the first sensitization day of oxazolone set as day 0, it was administered orally or transdermally starting on day 7 once a day. When the date of application of oxazolone and the date of administration of the drug were overlapped, the drug was administered about 6 hours after application of oxazolone.

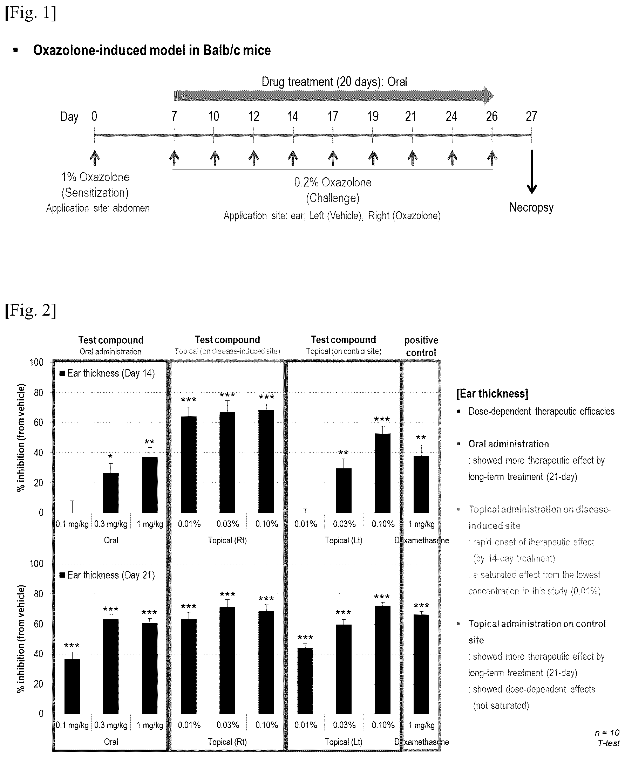

For all mice, abdominal hair was removed one day before the first application of oxazolone. Then, 1% or 0.2% oxazolone was prepared by the use of a 4:1 ratio of acetone/corn oil, and sensitization and induction on the shaved mouse abdomen and both ears were performed with the same dose and schedule as represented in Table 1 and Figure 1.

**[Table 1]**

| **Item** | **Schedule** | **Site** | **Application concentration and dose** |
|---|---|---|---|
| Sensitization | Day 0, 1 time | Abdomen | 1% oxazolone 50 µL |
| Challenge | (3 times a week, Mon/Wed/Fri) | Right ear | 0.2% oxazolone 25 µL |
| | | Left ear | 0.2% acetone/corn oil 25 µL |

### Example 2: Preparation of substances for oral administration

The test substances (the Test Compound and dexamethasone as a positive control) were prepared for each administration dose as represented in Table 2 below by the use of 0.5% methyl cellulose. In the case of the Test Compound, it was prepared in consideration of the HCl salt form. The test substances were dissolved in 0.5% methyl cellulose and then sonicated. If it did not completely dissolve, it was administered in a suspension state.

### Example 3: Preparation of substances for transdermal (topical) administration

The test substances were prepared by dissolving them at the concentration of 0.1% (w/v) in a solution (PEG400:100% ethanol = 1:1). 5 mL of PEG400 was added to 10 mg of the test substances and sonicated. When they were dissolved to some extent, 5 mL of 100% ethanol was added thereto and sonicated to dissolve them completely. In addition, the vehicle and the test substances were prepared fresh every day and used.

**[Table 2]**

| **Test Compound** | | **Dose** | | | **Vehicle application ear (Left, control site)** | **Oxazolone application ear (Right, diseaseinduced site)** |
|---|---|---|---|---|---|---|
| Oral administration | | 0.1 mg/kg (1.8 µg) | 0.3 mg/kg (5.4 µg) | 1 mg/kg (18 µg) | - | - |
| Topical administration | Disease-induced site | 0.01% (2.5 µg) | 0.03% (7.5 µg) | 0.1% (25 µg) | - | Topical administration |
| | Control site | | | | Topical administration | - |

### Example 4: Results of measurement

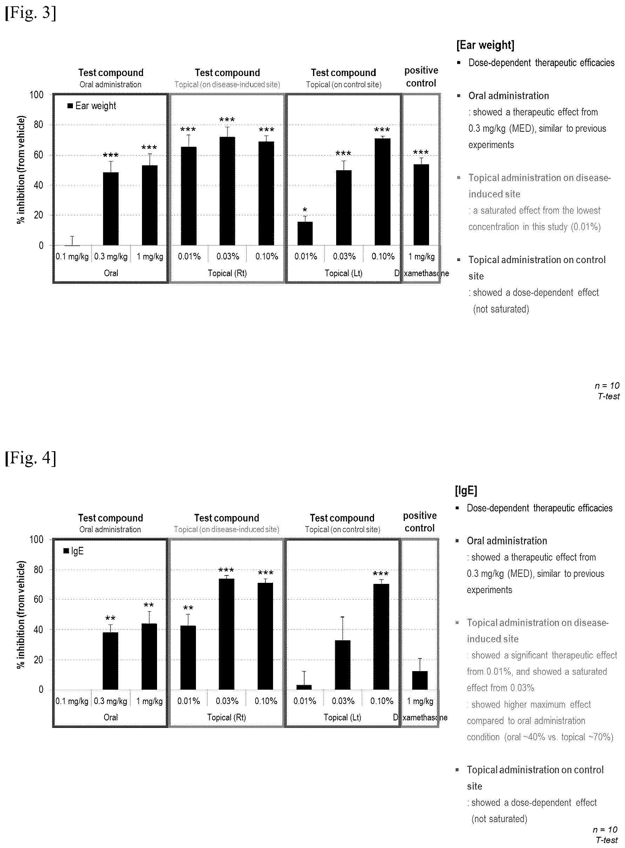

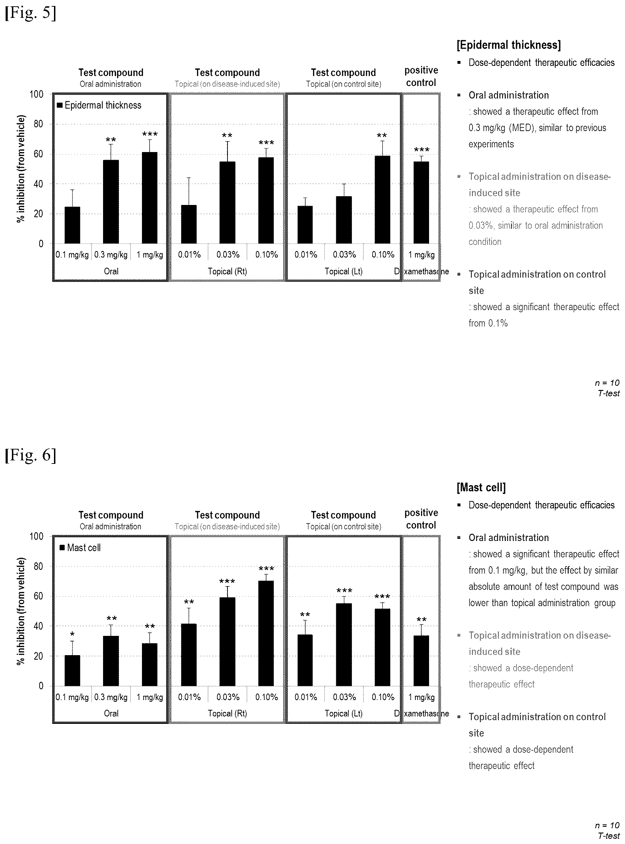

The reduction in ear thickness was measured on days 14 and 21 after administration of the test substances, and the results are represented in Table 3 and Figure 2. On the 21^{st} day after administration of the test substances, the reduction in ear weight was measured after autopsy, and the results are represented in Table 3 and Figure 3. The reduction in the blood level of IgE was measured on day 21 after administration of the test substances, and the results are represented in Table 3 and Figure 4. In addition, the reductions in epidermal thickness of the ear tissue and mast cell accumulation were measured on day 21 after administration of the test substances, and the results are represented in Table 3, Figures 5 and 6.

**[Table 3]**

| **Therapeutic effect (Compared to disease-induced group)** | **Test Compound oral administration** | | | **Test Compound topical administration** | | | **Positive control (Dexamethasone)** |
|---|---|---|---|---|---|---|---|
| | **0.1 mg/kg** | **0.3 mg/kg** | **1 mg/kg** | **0.01%** | **0.03%** | **0.1%** | **1 mg/kg** |
| **Ear thickness (Day 21 after administration)** | 37% | 63% | 61% | 63% | 71% | 69% | 66% |
| **Ear weight (Day 21 after administration)** | -2% | 48% | 53% | 65% | 72% | 69% | 54% |
| **Epidermal thickness of ear tissue (Day 21 after administration)** | 25% | 56% | 61% | 26% | 55% | 58% | 55% |
| **IgE (Day 21 after administration)** | -56% | 38% | 44% | 43% | 74% | 71% | 12% |
| **Mast cell accumulation (Day 21 after administration)** | 21% | 34% | 29% | 42% | 59% | 70% | 34% |

As can be seen from Table 3 and Figures 2 to 6, it can be known that the Test Compound showed superior effect in a dose-dependent manner compared to dexamethasone (positive control) in an oxazolone-induced animal model of atopic dermatitis. From such results, it was confirmed that the compound of the present invention can be used as a drug for the prevention or treatment of atopic dermatitis.

### Example 5: Comparative Experimentation

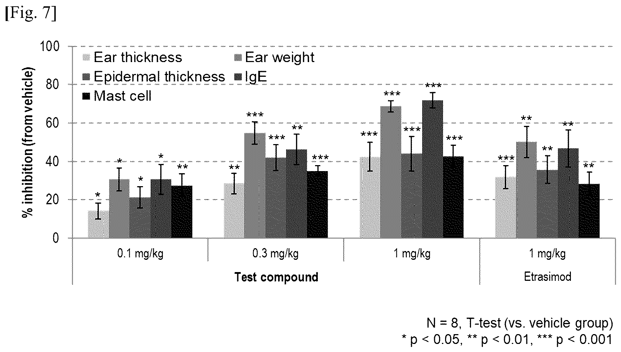

For comparison with another S1P receptor agonist, etrasimod (2-[(3R)-7-[[4-cyclopentyl-3-(trifluoromethyl)phenyl]methoxy]-1,2,3,4-tetrahydrocyclopenta[b]indol-3-yl]acetic acid), the reductions in ear thickness, ear weight reduction, blood level of IgE, epithelial thickness and mast cell accumulation were measured in the same manner as in Example 4, and the results are represented in Table 4 and Figure 7.

**[Table 4]**

| **Therapeutic effect (Compared to disease-induced group)** | **Test Compound oral administration** | | | **Comparative compound (etrasimod) oral administration** |
|---|---|---|---|---|
| | 0.1 mg/kg | 0.3 mg/kg | 1 mg/kg | 1 mg/kg |
| **Ear thickness (Day 21 after administration)** | 14% | 29% | 42% | 32% |
| **Ear weight (Day 21 after administration)** | 31% | 55% | 69% | 50% |
| **Epidermal thickness of ear tissue (Day 21 after administration)** | 21% | 42% | 44% | 36% |
| **IgE (Day 21 after administration)** | 31% | 46% | 72% | 47% |
| **Mast cell accumulation (Day 21 after administration)** | 27% | 35% | 43% | 28% |

As can be seen from Table 4 and Figure 7, it can be known that the Test Compound exhibited equal or superior effects even at lower doses compared to the comparative compound, etrasimod. From such results, it can be expected that side effects due to systemic exposure can be minimized through the administration of the same dose to obtain better efficacy or the administration of a lower dose to achieve a specific level of efficacy. In addition, superiority can be expected in terms of side effects through low affinity for GIRKs (G protein-coupled inwardly-rectifying potassium channels), which can induce brachycardia by S1P receptor agonists.

## Claims

1. A medicament for the prevention or treatment of atopic dermatitis comprising a therapeutically effective amount of a compound of the following Formula 1 or a pharmaceutically acceptable salt thereof: 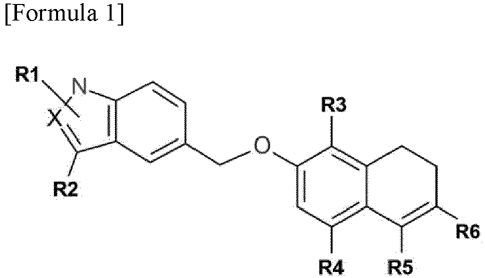 wherein
X is carbon or nitrogen;
R1 is hydrogen or alkyl;
R2 is hydrogen, alkyl, halogen, CN, CF₃ or COCF₃;
R3 and R4 are each independently hydrogen, alkyl, halogen, haloalkyl or alkoxyalkyl;
R5 is hydrogen, alkyl or halogen;
R6 is 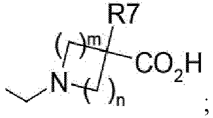
R7 is hydrogen or alkyl; and
m and n are each independently 0, 1, 2 or 3.

2. The medicament for the prevention or treatment of atopic dermatitis according to Claim 1, wherein
X is CH or N;
R1 is hydrogen or C₁-C₅ alkyl;
R2 is hydrogen, C₁-C₅ alkyl, halogen, CN, CF₃ or COCF₃;
R3 and R4 are each independently hydrogen, C₁-C₅ alkyl, halogen, halo-C₁-C₅ alkyl or C₁-C₅ alkoxy-C₁-C₅ alkyl;
R5 is hydrogen, C₁-C₅ alkyl or halogen;
R6 is 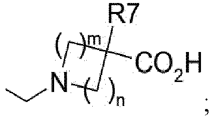
R7 is hydrogen or C₁-C₅ alkyl; and
m and n are each independently 0, 1, 2 or 3.

3. The medicament for the prevention or treatment of atopic dermatitis according to Claim 1, wherein the compound of Formula 1 is 1-[1-chloro-6-(3-chloro-1-isopropyl-1H-indazol-5-ylmethoxy)-3,4-dihydro-naphthalen-2-ylmethyl]-piperidine-4-carboxylic acid of the following Formula 2:

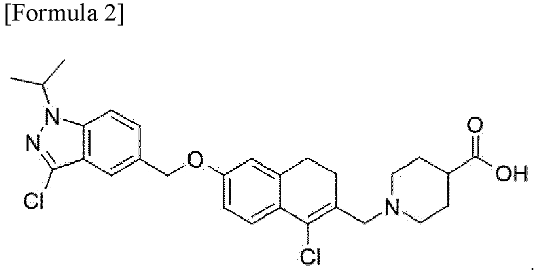

4. The medicament for the prevention or treatment of atopic dermatitis according to Claim 1, wherein the pharmaceutically acceptable salt is selected from the group consisting of hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, fumaric acid, maleic acid, methanesulfonic acid, benzenesulfonic acid, p-toluenesulfonic acid and naphthalenesulfonic acid.

5. A pharmaceutical composition for the prevention or treatment of atopic dermatitis comprising a therapeutically effective amount of a compound of the following Formula 1 or a pharmaceutically acceptable salt thereof, together with a pharmaceutically acceptable carrier: 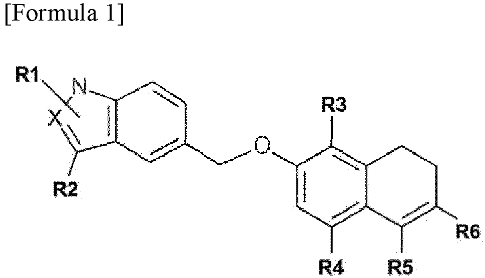 wherein
X is carbon or nitrogen;
R1 is hydrogen or alkyl;
R2 is hydrogen, alkyl, halogen, CN, CF₃ or COCF₃;
R3 and R4 are each independently hydrogen, alkyl, halogen, haloalkyl or alkoxyalkyl;
R5 is hydrogen, alkyl or halogen;
R6 is 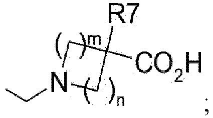
R7 is hydrogen or alkyl; and
m and n are each independently 0, 1, 2 or 3.

6. The pharmaceutical composition for the prevention or treatment of atopic dermatitis according to Claim 5, wherein
X is CH or N;
R1 is hydrogen or C₁-C₅ alkyl;
R2 is hydrogen, C₁-C₅ alkyl, halogen, CN, CF₃ or COCF₃;
R3 and R4 are each independently hydrogen, C₁-C₅ alkyl, halogen, halo-C₁-C₅ alkyl or C₁-C₅ alkoxy-C₁-C₅ alkyl;
R5 is hydrogen, C₁-C₅ alkyl or halogen;
R6 is 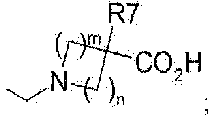
R7 is hydrogen or C₁-C₅ alkyl; and
m and n are each independently 0, 1, 2 or 3.

7. The pharmaceutical composition for the prevention or treatment of atopic dermatitis according to Claim 5, wherein the compound of Formula 1 is 1-[1-chloro-6-(3-chloro-1-isopropyl-1H-indazol-5-ylmethoxy)-3,4-dihydro-naphthalen-2-ylmethyl]-piperidine-4-carboxylic acid of the following Formula 2:

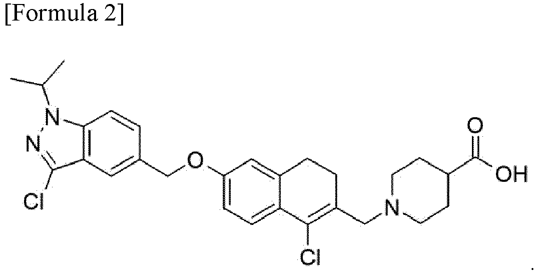

8. The pharmaceutical composition for the prevention or treatment of atopic dermatitis according to Claim 5, wherein the pharmaceutically acceptable salt is selected from the group consisting of hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, fumaric acid, maleic acid, methanesulfonic acid, benzenesulfonic acid, p-toluenesulfonic acid and naphthalenesulfonic acid.

9. A method for the prevention or treatment of atopic dermatitis comprising administering a therapeutically effective amount of a compound of the following Formula 1 or a pharmaceutically acceptable salt thereof to a subject in need thereof: 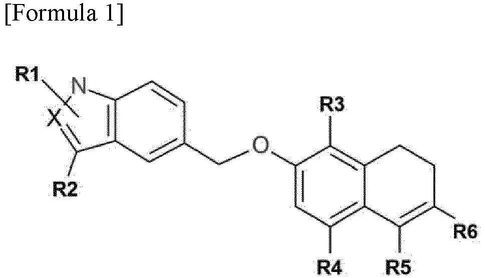 wherein
X is carbon or nitrogen;
R1 is hydrogen or alkyl;
R2 is hydrogen, alkyl, halogen, CN, CF₃ or COCF₃;
R3 and R4 are each independently hydrogen, alkyl, halogen, haloalkyl or alkoxyalkyl;
R5 is hydrogen, alkyl or halogen;
R6 is 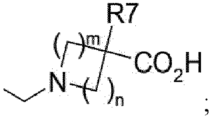
R7 is hydrogen or alkyl; and
m and n are each independently 0, 1, 2 or 3.

10. Use of a compound of the following Formula 1 or a pharmaceutically acceptable salt thereof in the manufacture of a medicament for the prevention or treatment of atopic dermatitis: 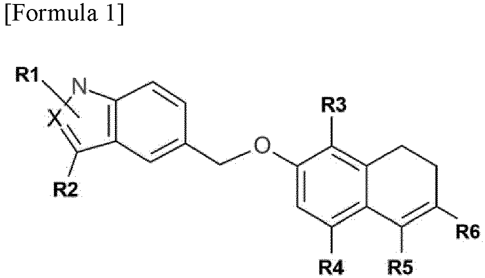 wherein
X is carbon or nitrogen;
R1 is hydrogen or alkyl;
R2 is hydrogen, alkyl, halogen, CN, CF₃ or COCF₃;
R3 and R4 are each independently hydrogen, alkyl, halogen, haloalkyl or alkoxyalkyl;
R5 is hydrogen, alkyl or halogen;
R6 is 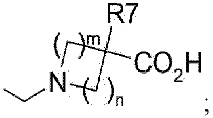
R7 is hydrogen or alkyl; and
m and n are each independently 0, 1, 2 or 3.
